# EUROPÄISCHE PATENTANMELDUNG

(11) **EP 3 238 662 A1**
(43) Veröffentlichungstag der Anmeldung: **01.11.2017**
(21) Anmeldenummer: 17164727.4
(22) Anmeldetag: 04.04.2017
(51) Int. Cl.: A61F 2/24

(54) **HERZKLAPPENPROTHESE**

(30) Priorität: 11.04.2016 DE 102016106575
(71) Anmelder: Biotronik AG, 8180 Bülach (CH)
(72) Erfinder: Ulmer, Jens, 8700 Küsnacht (CH); Degen, Nicolas, 8222 Beringen (CH)
(74) Vertreter: Galander, Marcus

(57) **Zusammenfassung**

Die Erfindung betrifft Herzklappenprothese (1), mit: einem Stentgerüst (2), das von einem kollabierten Zustand in einen expandierten Zustand überführbar ist, in dem sich das Stentgerüst (2) entlang einer Achse (A') erstreckt, wobei das Stentgerüst (2) eine Mehrzahl an Streben (20, 24) aufweist, die eine Mehrzahl an miteinander verbundenen Zellen (21a, 21b, 25) ausbilden, und einer Herzklappe (3), die am Stentgerüst (2) festgelegt ist. Erfindungsgemäß ist vorgesehen, dass die Dicke (d') der Streben in Umfangsrichtung (U) des expandierten Stentgerüsts (2) variiert.

## Beschreibung

Die Erfindung betrifft eine Herzklappenprothese, insbesondere zum transcutanen Ersatz einer Herzklappe, vorzugsweise Mitralklappe, gemäß Anspruch 1.

Derartige Herzklappenprothesen weisen in der Regel ein Stentgerüst auf, das von einem kollabierten Zustand in einen expandierten Zustand überführbar ist und eine Mehrzahl an Streben aufweist, die eine Mehrzahl an miteinander verbundenen Zellen ausbilden, sowie ferner einer Herzklappe (insbesondere aus einem biologischen Gewebe), die am Stentgerüst festgelegt ist.

Bei der Implantation von künstlichen Herzklappen, insbesondere einer Mitralklappe ist es für die korrekte und dauerhafte Funktion dieser künstlichen Herzklappe notwendig, diese den anatomischen Gegebenheiten im Bereich der natürlichen Herzklappe (Anulus) anzupassen. So ist es z.B. notwendig, diese Klappe in Ihrer radialen Geometrie asymmetrisch zu gestalten. Dabei ist insbesondere auf die D-förmige Anulusgeometrie der nativen Klappe Rücksicht zu nehmen.

Der Erfindung liegt hiervon ausgehend die Aufgabe zugrunde eine Herzklappenprothese bereitzustellen, die eine derartige Anpassung ermöglicht.

Diese Aufgabe wird durch eine Herzklappenprothese mit den Merkmalen des Anspruchs 1 gelöst. Vorteilhafte Ausgestaltungen der Erfindung sind in den Unteransprüchen angegeben und werden nachfolgend beschrieben.

Gemäß Anspruch 1 ist erfindungsgemäß vorgesehen, dass die Dicke der Streben in Umfangsrichtung des Stentgerüsts variiert.

Hierbei erstreckt sich vorzugsweise das Stentgerüst im expandierten Zustand entlang einer Achse, entlang der das Blut des Empfängers der Prothese durch einen vom Stentgerüst definierten bzw. umgebenen Innenraum des Stentgerüsts strömt, wenn die Prothese bestimmungsgemäß implantiert wurde, wobei das Stentgerüst in der senkrecht zu jener Achse umlaufenden Umfangsrichtung des Stentgerüsts umläuft.

Gemäß einer besonders bevorzugten Ausführungsform der Erfindung ist vorgesehen, dass die Herzklappenprothese dazu konfiguriert ist, die Funktion einer nativen Herzklappe zu übernehmen, wobei die besagte Herzklappe der Herzklappenprothese eine Mitralklappe ist.

Gemäß einer besonders bevorzugten Ausführungsform der Erfindung ist weiterhin vorgesehen, dass die Dicke der Streben in Umfangsrichtung variiert, derart, dass das Stentgerüst im expandierten und implantierten Zustand eine umlaufende Querschnittskontur aufweist, die dem Mitralklappenanulus der zu ersetzenden Mitralklappe angepasst ist, wobei jene Querschnittkontur insbesondere D-förmig ist.

Mit anderen Worten weist die Querschnittskontur gemäß einer Ausführungsform der Erfindung im implantierten Zustand einen abgeflachten ersten Abschnitt und einem damit verbundenen bogenförmigen zweiten Abschnitt auf, wobei der sich der abgeflachten Abschnitt bezogen auf den expandierten sowie implantierten Zustand des Stentgerüsts vom Bereich des linken fibrösen Trigonums zum Bereich des rechten fibrösen Trigonums erstreckt.

Gemäß einer bevorzugten Ausführungsform der Erfindung ist weiterhin vorgesehen, dass die Geometrie der Zellen des Stentgerüstes in Umfangsrichtung variiert, derart, dass das Stentgerüst im expandierten und implantierten Zustand eine umlaufende Querschnittskontur aufweist, die dem Mitralklappenanulus der zu ersetzenden Mitralklappe angepasst ist, wobei jene Querschnittkontur insbesondere D-förmig ist.

Im Rahmen dieser Anmeldung werden für das Stentgerüst bevorzugt drei grundsätzliche Zustände unterschieden, der komprimierte Zustand, der expandierte Zustand und der implantierte Zustand (im Folgenden auch häufig als "expandierter sowie implantierter Zustand" bezeichnent). Der expandierte Zustand ist der Normalzustand des Stentgerüstes. Diesen frei, expandierten Zustand nimmt das Stentgerüst im freien Raum ohne jede Einwirkung äußerer Kräfte ein. Im komprimierten Zustand ist das Stentgerüst durch die Einwirkung äußerer Kräfte in seiner radialen Ausdehnung komprimiert. In diesem Zustand wird die Prothese zumeist in den Körper, insbesondere mittels eines Einführkatheters, eingeführt und an den Implantationsort transportiert. Mit Implantation der Prothese nimmt das Stentgerüst wieder seinen expandierten und jetzt implantierten Zustand ein. Der implantierte Zustand unterscheidet sich vom frei expandierten Zustand dadurch, dass sich das Stentgerüst in der äußeren Umgebung des Implantationsortes befindet und dieser entsprechend auf das expandierte Stentgerüst einwirkt.

Das Stentgerüst dieser bevorzugten Ausgestaltung würde in seiner reinen expandierten Form einen kreisförmigen Querschnitt einnehmen. Die erfindungsgemäße Variation der Dicke der Streben des Stentgerüstes erlaubt der Prothese bzw. dem Stentgerüst jedoch, sich dem natürlichen Querschnitt des Mitralklappenanuluses anzupassen und einen Querschnitt wie vorangehend beschrieben einzunehmen.

Gemäß einer besonders bevorzugten Ausführungsform der Erfindung ist vorgesehen, dass das Stentgerüst erste Streben aufweist, die bezogen auf den expandierten sowie implantierten Zustand des Stentgerüsts im Bereich des linken fibrösen Trigonums sowie im Bereich des rechten fibrösen Trigonums zu liegen kommen, wobei diese ersten Streben eine geringere Dicke aufweisen, als die zweite Streben des Stengerüsts, die weiter abseits des linken fibrösen Trigonums sowie des rechten fibrösen Trigonums auf dem Mitralklappenanulus zu liegen kommen.

Durch diese Ausgestaltung wird eine Querschnittskontur aus einem abgeflachten ersten Abschnitt und einem damit verbundenen bogenförmigen zweiten Abschnitt erreicht, wobei der sich der abgeflachten Abschnitt bezogen auf den expandierten sowie implantierten Zustand des Stentgerüsts vom linken fibrösen Trigonum zum rechten fibrösen Trigonum erstreckt. Durch die dünneren ersten Streben, die im implantierten Zustand im Bereich der Trigonen zu liegen kommen, wird ein "Einknicken" des Stents an dieser Stelle ermöglicht. Damit ergibt sich eine "D-förmige" Geometrie im implantierten Zustand, die der natürlichen Geometrie des Mitralklappenannulus entspricht. Eine Vorprägung einer derartigen Geometrie wie im Stand der Technik ist nicht notwendig.

Gemäß einer besonders bevorzugten Ausführungsform der Erfindung ist vorgesehen, dass das Stentgerüst erste Streben aufweist, die bezogen auf den expandierten sowie implantierten Zustand des Stentgerüsts im Bereich des linken fibrösen Trigonums sowie im Bereich des rechten fibrösen Trigonums zu liegen kommen, wobei das Stentgerüst eine in diesen Bereichen geänderte Stentstruktur aufweist, die in Folge zu einer lokalen Schwächung der radialen Kraft des Stentgerüst führt.
Gemäß einer besonders bevorzugten Ausführungsform der Erfindung ist vorgesehen, dass das Stentgerüst im Bereich des linken fibrösen Trigonums sowie im Bereich des rechten fibrösen Trigonums zu eine offene Zellenstrucktur aufweist, bei der zwei oder mehrere Zellen nicht miteinander verbunden sind.

Gemäß einer besonders bevorzugten Ausführungsform der Erfindung ist vorgesehen, dass das Stentgerüst im Bereich des linken fibrösen Trigonums sowie im Bereich des rechten fibrösen Trigonums zu eine geänderte Zellengeometrie aufweist, in der in diesen Bereichen die Größe und Anzahl der Zellen variiert.

Gemäß einer bevorzugten Ausführungsform der Erfindung ist vorgesehen, dass die ersten Streben eine Dicke aufweisen, die im Bereich des 0,5-fachen bis 0,9-fachen der Dicke der zweiten Streben liegt, wobei vorzugsweise die Dicke der ersten Streben das 0,7-fache bis 0,9-fache, insbesondere das 0,8-fache der Dicke der zweiten Streben beträgt.

Vorzugsweise sind die Streben des Stentgerüsts gemäß einer Ausführungsform der Erfindung über Verbindungsbereiche integral miteinander verbunden bzw. integral aneinander angeformt.

Gemäß einer bevorzugten Ausführungsform der Erfindung ist weiterhin vorgesehen, dass die ersten Streben zwei Zellen des Stentgerüstes bilden, die in Richtung der Achse des Stentgerüstes nebeneinander bzw. übereinander angeordnet sind und über einen Verbindungsbereich miteinander verbunden sind, wobei jene beiden Zellen bezogen auf den expandierten sowie implantierten Zustand des Stentgerüsts im Bereich des linken fibrösen Trigonums zu liegen kommen.

Gemäß einer bevorzugten Ausführungsform der Erfindung ist weiterhin vorgesehen, dass der Verbindungsbereich einen ersten und einen zweiten Randabschnitt aufweist, wobei die beiden Randabschnitte bei einem expandierten Stentgerüst in der Umfangsrichtung einander gegenüberliegen, wobei in jedem Randabschnitt eine Ausnehmung ausgebildet ist, und wobei die beiden Ausnehmungen in Richtung der besagten Achse des Stentgerüstes versetzt zueinander angeordnet sind.

Gemäß einer bevorzugten Ausführungsform der Erfindung ist in analoger Weise vorgesehen, dass die ersten Streben zwei weitere Zellen des Stentgerüstes bilden, die in Richtung der besagten Achse nebeneinander bzw. übereinander angeordnet sind und über einen weiteren Verbindungsbereich miteinander verbunden sind, wobei jene beiden weiteren Zellen bezogen auf den expandierten sowie implantierten Zustand des Stentgerüsts im Bereich des rechten fibrösen Trigonums zu liegen kommen.

Gemäß einer bevorzugten Ausführungsform der Erfindung ist weiterhin vorgesehen, dass der weitere Verbindungsbereich einen ersten und einen zweiten Randabschnitt aufweist, wobei die beiden Randabschnitte in der Umfangsrichtung einander gegenüberliegen, wobei in jedem Randabschnitt eine Ausnehmung ausgebildet ist, und wobei die beiden Ausnehmungen in Richtung der Achse versetzt zueinander angeordnet sind.

Die geringeren Dicken der Streben im Bereich des linken und rechten Trigonums sowie die oben beschriebene Ausdünnung der Verbindungsbereiche jener dünneren Streben bzw. Zellen ermöglichen bzw. begünstigen mit Vorteil eine Verformung des expandierten Stentgerüstes, bei der das Stentgerüst jene an den Mitralklappenanulus angepasste Geometrie bzw. Querschnittskontur aufweist (insbesondere D-förmige Querschnittskontur).

Gemäß einer bevorzugten Ausführungsform der Erfindung ist weiterhin vorgesehen, dass das Stentgerüst einen umlaufenden ersten Randbereich aufweist, der einen Einströmtrakt der Herzklappenprothese umgibt, über den Blut in die Herzklappenprothese einströmen kann sowie einen gegenüberliegenden umlaufenden zweiten Randbereich, der einen Ausströmtrakt der Herzklappenprothese definiert, über den Blut aus der Herzklappenprothese ausströmen kann.

Gemäß einer bevorzugten Ausführungsform der Erfindung ist weiterhin vorgesehen, dass das Stentgerüst am Ausströmtrakt Bügel zum Verankern der Herzklappenprothese an den Segeln der nativen Mitralklappe aufweist.

Gemäß einer bevorzugten Ausführungsform der Erfindung sind diesbezüglich vorzugsweise zwei Bügel vorgesehen, die dazu konfiguriert sind, die Herzklappenprothese am anterioren Segel der nativen Mitralklappe zu verankern.

Hierbei ist bevorzugt vorgesehen, dass diese beiden Bügel jeweils ausgehend vom Stentgerüst auseinanderlaufen, wobei jeder Bügel in Richtung eines zugeordneten Trigonums der nativen Mitralklappe verläuft.

Gemäß einer weiteren Ausführungsform der Erfindung ist bevorzugt (insbesondere lediglich) ein weiterer Bügel vorgesehen, der dazu konfiguriert ist, die Herzklappenprothese am posterioren Segel der nativen Mitralklappe zu verankern, wobei der weitere Bügel in seiner Erstreckungsrichtung kürzer ausgestalt ist als die beiden Bügel für das anteriore Segel. Vorzugsweise beträgt die Länge dieses weiteren Bügels das 0,7- bis 0,8-fache, vorzugsweise das 0,75-fache der Länge der beiden anderen Bügel.

Gemäß einer bevorzugten Ausführungsform der Erfindung ist weiterhin vorgesehen, dass das Stentgerüst zur Verankerung der Herzklappenprothese am Ausströmtrakt Zellen aufweist, die nach außen gebogen sind.

Gemäß einer bevorzugten Ausführungsform der Erfindung ist weiterhin vorgesehen, dass das Stentgerüst an einem umlaufenden Rand eines Einströmtrakts des Stentgerüsts verkürzte Zellen aufweist, deren Länge in Richtung der besagten Achse des (expandierten) Stentgerüstes kürzer ist als die Länge von in der Umfangsrichtung benachbarten Zellen des Stentgerüstes, wobei die verkürzten Zellen dazu konfiguriert sind, im expandierten sowie implantierten Zustand des Stentgerüsts im Bereich des Aortenpeaks der nativen Mitralklappe zum liegen zu kommen.

Gemäß einer bevorzugten Ausführungsform der Erfindung ist weiterhin vorgesehen, dass die Herzklappenprothese dazu konfiguriert ist, minimalinvasiv mittels eines Katheters implantiert zu werden.

Gemäß einer bevorzugten Ausführungsform der Erfindung ist weiterhin vorgesehen, dass das Stentgerüst expandierbar (z.B. mittels eines Ballons), vorzugsweise selbstexpandierend ausgebildet ist. In letzterem Fall entfaltet sich das Stentgerüst selbsttätig, sobald es durch den zur Implantation verwendeten Katheter freigegeben wird, der das Stentgerüst im komprimierten Zustand zum Implantationsort transportiert.

Weitere Merkmale und Vorteile der Erfindung sollen bei der Figurenbeschreibung von Ausführungsbeispielen der Erfindung anhand der Figuren erläutert werden. Es zeigen:
- Fig. 1: schematische Ansichten einer Mitralklappe;
- Fig. 2: eine Stentgerüst einer erfindungsgemäßen Herzklappenprothese in flach ausge-breiteten Zustand;
- Fig. 3: eine schematische Darstellung einer erfindungsgemäßen Herzklappenprothese in einem implantierten Zustand;
- Fig. 4: eine weitere schematische Darstellung einer erfindungsgemäßen Herzklappenprothese in einem implantierten Zustand;
- Fig. 5: eine schematische Ansicht einer erfindungsgemäßen Herzklappenprothese; und

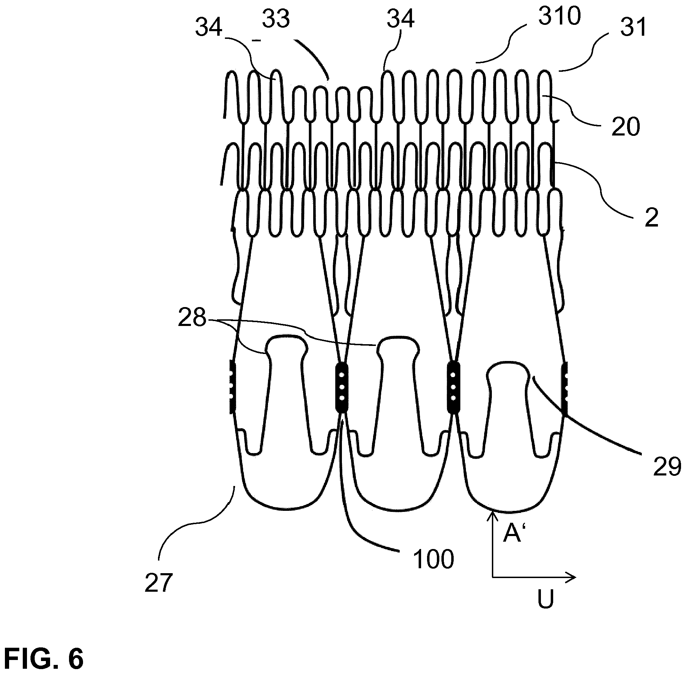

- Fig. 6: eine schematische Ansicht eines weiteren Stentgerüstes einer erfindungsgemäßen Herzklappenprothese in einem flach ausgebreiteten Zustand.

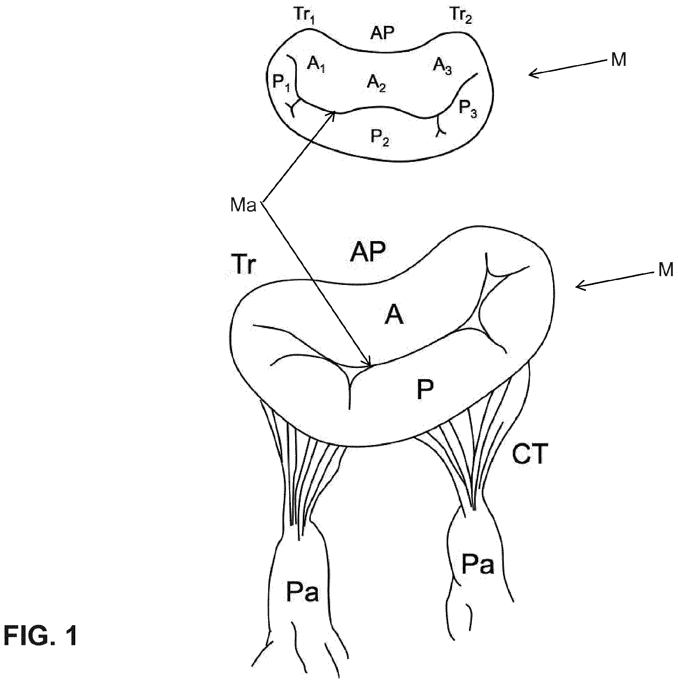

Figur 1 zeigt Ansichten eine Mitralklappe M. Die erfindungsgemäße Herzklappenprothese 1 ist insbesondere dazu konfiguriert, eine derartige defekte Klappe M zu ersetzen, wobei diese durch ein Stentgerüst 2 der Herzklappenprothese lateral verdrängt wird. Der entsprechend sich ausbildende Mitralklappenanulus Ma hat eine D-förmige Geometrie, wobei das Stentgerüst 2 durch das Vorsehen von insbesondere dünneren Streben 20 die besagte Geometrie im Bereich des Anulus Ma erhält. Im Einzelnen bezeichnen A1 bis A3 die Abschnitte des anterioren Klappensegels A, während P1 bis P3 die Abschnitte des posterioren Klappensegel P bezeichnen. Weiterhin sind der "Aortic peak" AP sowie das linke und rechte fibröse Trigonum Tr1, Tr2 eingetragen.

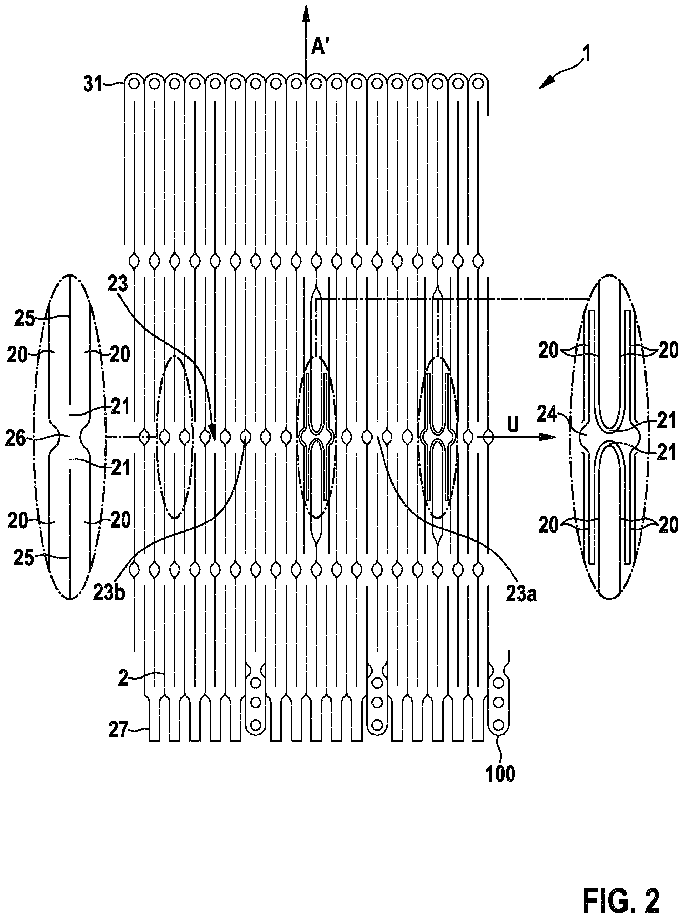

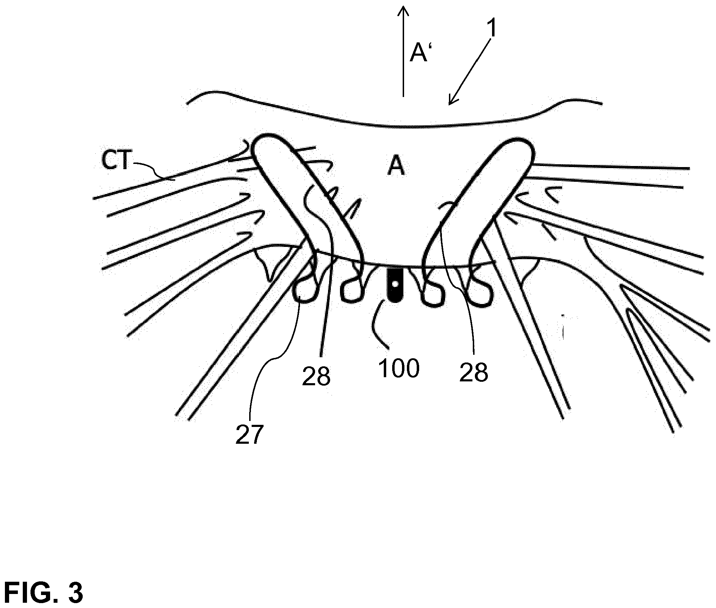

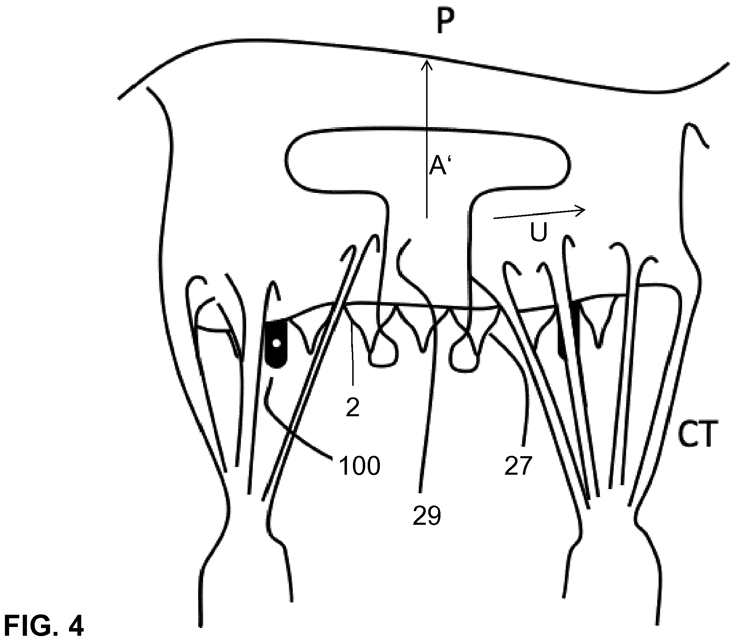

In Fig. 2 ist ein Schnittmuster eines erfindungsgemäßen Stentgerüstes 2 für den Aufbau einer Mitralklappenprothese 1 dargestellt. Das Stentgerüst 2 weist drei Anker 100 für die Befestigung der Kommisuren der künstlichen Klappensegel auf, die hier nicht dargestellt sind sowie vorzugsweise insgesamt 18 radial umlaufende Zellen 20 bzw. 24, wobei der untere Teil der Stentzellen 20 mit Fortsätzen versehen werden kann, an die zusätzliche Haltebügel 28, 29 (vgl. z.B. siehe Abb.3 und 4) angebracht werden können. Insgesamt sind die Stentzellen 20, 24 in dieser Ausgestaltung insbesondere in vier Reihen übereinander angeordnet und bilden so das gesamte Stentgerüst 2. Entscheidend für den Erhalt einer D-förmigen Geometrie sind, wie eingangs dargelegt, unterschiedlich dicke Streben 20, 24, die nach der Implantation im Bereich der Trigonen Tr1, Tr2 (siehe auch Fig. 1) zu liegen kommen, bei denen je ein Bereich der größten Biegung der Querschnittkontur 23 des expandierten Stentgerüstes 2 vorliegt (im implantierten sowie expandierten Zustand ist der linke vertikale Rand des Schnittmusters 2 mit dem rechten Rand des Schnittmusters 2 verbunden, so dass entsprechend ein Stentgerüst 2 ausgebildet wird, das sich entlang einer zentralen Achse A' erstreckt und quer zu dieser in einer Umfangsrichtung U umläuft, wobei das Stentgerüst 2 (bezogen auf die Achse A') am oberen und unteren Ende einen Einströmtrakt 31 bzw. einen Ausströmtrakt 27 ausbildet.

Um eine geeignete Krümmung des Stentgerüstes 2 zu erreichen, kann z.B. die Stentdicke d' der ersten Stents 20 gegenüber den zweiten Stents 24 0,8*d betragen, wobei d die Strebendicke der Streben 24 in allen anderen Bereichen (abseits der Trigonen Tr1, Tr2) angibt. Zusätzlich zu den dünneren Stentstreben 20 werden die Verbindungsbereiche 26b bzw. 26a (nicht gezeigt) der einzelnen (dünneren) Stentzellen 21a, 21b flexibel ausgestaltet. Dies erlaubt es, dass die Stentgeometrie besser der natürlichen Mitralgeometrie folgen kann und sich so die künstliche Herzklappe besser im Anulus Ma verankert.

Vorzugsweise ist hierzu z.B. vorgesehen, dass der jeweilige Verbindungsbereich 26b (bzw. 26a) der dünneren Stents 20 einen ersten und einen zweiten Randabschnitt 210, 211 aufweist, wobei die beiden Randabschnitte 210, 211 in der Umfangsrichtung U des Stentgerüstes 2 einander gegenüberliegen, wobei in jedem Randabschnitt 210, 211 eine Ausnehmung 212 ausgebildet ist, wobei die beiden Ausnehmungen 212 in Richtung der Achse A' des Stentgerüstes 2 versetzt zueinander angeordnet sind.

Für die Fixierung der Klappe 1 werden vorzugsweise weiterhin am Ausströmtrakt 27 Bügelstrukturen 28 angebracht. Dabei werden für die Fixierung des anterioren Segels A zwei Bügel 28 z.B. nach Fig. 3 angebracht. Dabei verlaufen die Bügel 28 von der Kommissuraufhängung 100 in Richtung der Trigonen Tr1, Tr2, um sich dort abstützen zu können. Diese schräg verlaufende Bügel 28 sollen insbesondere ein Verhaken mit den Chordae Tendineae CT während der Implantation verringern. Im Bereich des posterioren Segels P wird nur ein Bügel 29 vorgesehen (vgl. Fig. 4). Dieser ist vorzugsweise so gestaltet, dass er im distalen Bügelbereich eine möglichst große Fläche des Segels P bedeckt ohne sich dabei während der Positionierung des Bügels 29 mit den Chordae Tendineae CT zu verhaken. Weiterhin ist der posteriore Bügel 29 vorzugsweise verkürzt ausgestaltet und entspricht in einem Beispiel ungefähr dem 0,75 fachen der Länge der anterioren Bügel 28.

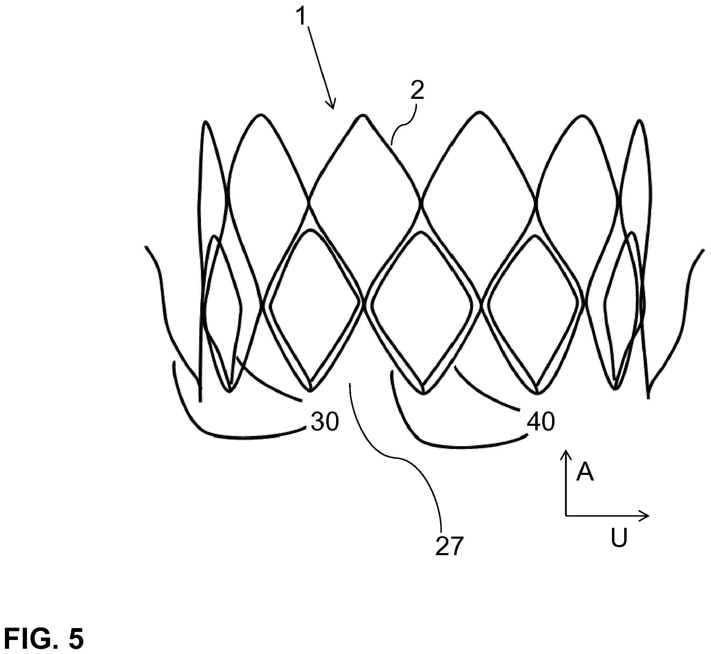

Bedeutsam für die Fixierung der künstlichen Mitralklappe 1 ist die Verankerung mit den nativen Segeln (in Fig. 1 mit A für anteriores und mit P für posteriores Segel bezeichnet). Dies kann im Allgemeinen durch Verwendung von großen Bügeln (siehe auch Fig. 3 und 4), welche im Ausströmtrakt 27 der Herzklappe 1 angebracht sind, erfolgen. Weiterhin ist es gemäß Fig. 5 möglich, die Zellengeometrie so auszugestalten, dass ein Teil 30 der zellenbildenden Streben 40 nach außen gebogen werden kann. Der restliche Teil der Streben 40 bildet dann die jeweilige Zelle selbst. So ist es möglich im Ausströmtrakt 27 des Stentgerüsts 2 einen zusätzlichen Verankerungsmechanismus zu verwirklichen, der die künstliche Klappe 1 vor einer möglichen Dislokation bewahrt.

In Fig .6 ist ein weiteres Schnittmuster eines Stentgerüsts 2 gezeigt, welches den asymmetrischen Gegebenheiten im Bereich des Mitralanullus angepasst wurde (z.B. durch Verwendung der oben beschriebenen dünneren Streben 20). So sind weiterhin die Zellen 33 am Rand 310 des Einströmtrakts 31 im Bereich des Aortenpeaks AP (vergleiche Fig. 1) verkürzt ausgestaltet im Vergleich zu beidseitig benachbarten Zellen 34. Weiterhin haben die integrierten Haltebügel 28, 29 wiederum eine unterschiedliche Länge in Richtung der Achse A'. So ist der Haltebügel 29 des posterioren Segels P kürzer ausgestaltet als die beiden Haltebügel 28 des anterioren Segels A. Der posteriore Bügel 29 ist dabei z.B. 0,75-mal so lang wie der jeweilige anteriore Bügel 28. Hierbei ist zu Beachten, dass dem Stentgerüst 2 in der Fig. 4 auch beim heatsetting eine asymmetrische D-Form aufgeprägt werden kann. Im Gegensatz dazu kann das Stentgerüst 2 in Fig. 2 eine radialsymmetrische Ausgangs-form im reinen expandierten Zustand aufweisen, wobei es sich aufgrund der unterschiedlichen mechanischen Eigenschaften des Stentgerüsts 2 (bedingt durch die verschieden dicken Streben 20, 24 bzw. die Verbindungsbereiche 26a, 26b) an die D-Form des natürlichen Mitralanulus Ma anpassen kann.

## Patentansprüche

1. Herzklappenprothese (1), mit:
- einem Stentgerüst (2), das von einem kollabierten Zustand in einen expandierten Zustand überführbar ist, in dem sich das Stentgerüst (2) entlang einer Achse (A') erstreckt, wobei das Stentgerüst (2) eine Mehrzahl an Streben (20, 24) aufweist, die eine Mehrzahl an miteinander verbundenen Zellen (21a, 21b, 25) ausbilden, und
- einer Herzklappe (3), die am Stentgerüst (2) befestigt ist,
**dadurch gekennzeichnet,**
**dass** die Dicke (d') der Streben in Umfangsrichtung (U) variiert.

2. Herzklappenprothese nach Anspruch 1, **dadurch gekennzeichnet, dass** die Herzklappenprothese (1) dazu konfiguriert ist, die Funktion einer nativen Mitralklappe (M) zu übernehmen, wobei die besagte Herzklappe (3) der Herzklappenprothese (1) eine Mitralklappe (3) ist.

3. Herzklappenprothese nach Anspruch 1 oder 2, **dadurch gekennzeichnet, dass** die Dicke (d, d') der Streben (20, 24) in Umfangsrichtung (U) variiert, derart, dass das Stentgerüst (2) im expandierten und implantierten Zustand eine umlaufende Querschnittskontur (23) aufweist, die dem Mitralklappenanulus (Ma) der zu ersetzenden Mitralklappe (M) angepasst ist.

4. Herzklappenprothese nach Anspruch 3, **dadurch gekennzeichnet, dass** die Querschnittskontur (23) aus einem abgeflachten ersten Abschnitt (23a) und einem damit verbundenen bogenförmigen zweiten Abschnitt (23b) besteht, wobei der sich der abgeflachten Abschnitt (23a) bezogen auf den expandierten sowie implantierten Zustand des Stentgerüsts (2) vom linken fibrösen Trigonum (Tr1) zum rechten fibrösen Trigonum (Tr2) erstreckt.

5. Herzklappenprothese nach einem der vorhergehenden Ansprüche, **dadurch gekennzeichnet, dass** das Stentgerüst (2) erste Streben (20) aufweist, die bezogen auf den expandierten sowie implantierten Zustand des Stentgerüsts (2) im Bereich des linken fibrösen Trigonums (Tr1) sowie im Bereich des rechten fibrösen Trigonums (Tr2) zu liegen kommen, wobei diese ersten Streben (20) eine geringere Dicke (d') aufweisen, als zweite Streben (24) des Stentgerüsts (2), die weiter abseits des linken fibrösen Trigonums (Tr1) sowie des rechten fibrösen Trigonums (Tr2) auf dem Mitralklappenanulus (Ma) zu liegen kommen.

6. Herzklappenprothese nach Anspruch 5, **dadurch gekennzeichnet, dass** die ersten Streben (20) ein Dicke (d') aufweisen, die im Bereich des 0,5-fachen bis 0,9-fachen der Dicke (d) der zweiten Streben (24) liegt, wobei vorzugsweise die Dicke (d') der ersten Streben (20) das 0,8-fache der Dicke (d) der zweiten Streben (24) beträgt.

7. Herzklappenprothese nach Anspruch 5 oder 6, **dadurch gekennzeichnet, dass** die ersten Streben (20) zwei Zellen (21a) des Stentgerüstes bilden, die in Richtung der Achse (A') nebeneinander angeordnet sind und über einen Verbindungsbereich (26a) miteinander verbunden sind, wobei jene beiden Zellen (21a) bezogen auf den expandierten sowie implantierten Zustand des Stentgerüsts (2) im Bereich des linken fibrösen Trigonums (Tr1) zu liegen kommen.

8. Herzklappenprothese nach Anspruch 7, **dadurch gekennzeichnet, dass** der Verbindungsbereich (26a) einen ersten und einen zweiten Randabschnitt (210, 211) aufweist, wobei die beiden Randabschnitte (210, 211) in der Umfangsrichtung (U) einander gegenüberliegen, wobei in jedem Randabschnitt (210, 211) eine Ausnehmung (212) ausgebildet ist, und wobei die beiden Ausnehmungen (212) in Richtung der Achse (A) versetzt zueinander angeordnet sind.

9. Herzklappenprothese nach einem der Ansprüche 5 bis 8, **dadurch gekennzeichnet, dass** die ersten Streben (20) zwei weitere Zellen (21b) des Stentgerüstes (2) bilden, die in Richtung der Achse (A') nebeneinander angeordnet sind und über einen weiteren Verbindungsbereich (26b) miteinander verbunden sind, wobei jene beiden weiteren Zellen (21b) bezogen auf den expandierten sowie implantierten Zustand des Stentgerüsts (2) im Bereich des rechten fibrösen Trigonums (Tr2) zu liegen kommen.

10. Herzklappenprothese nach Anspruch 9, **dadurch gekennzeichnet, dass** der weitere Verbindungsbereich (26b) einen ersten und einen zweiten Randabschnitt (210, 211) aufweist, wobei die beiden Randabschnitte (210, 211) in der Umfangsrichtung (U) einander gegenüberliegen, wobei in jedem Randabschnitt (210, 211) eine Ausnehmung (212) ausgebildet ist, und wobei die beiden Ausnehmungen (212) in Richtung der Achse (A') versetzt zueinander angeordnet sind.

11. Herzklappenprothese nach einem der vorhergehenden Ansprüche, **dadurch gekennzeichnet, dass** das Stentgerüst (2) zum Ausströmen von Blut einen Ausströmtrakt (27) definiert, wobei das Stentgerüst (2) am Ausströmtrakt (27) Bügel (28, 29) zum Verankern der Herzklappenprothese (1) an den Segeln (A, P) der nativen Mitralklappe (M) aufweist, wobei bevorzugt zwei Bügel (28) vorgesehen sind, die dazu konfiguriert sind, die Herzklappenprothese (1) am anterioren Segel (A) der nativen Mitralklappe (M) zu verankern, und wobei bevorzugt ein weiterer Bügel (29) vorgesehen ist, der dazu konfiguriert ist, die Herzklappenprothese (1) am posterioren Segel (P) der nativen Mitralklappe (M) zu verankern, wobei der weitere Bügel (29) kürzer ausgestalt ist, als die beiden Bügel (28) für das anteriore Segel (A).

12. Herzklappenprothese nach einem der vorhergehenden Ansprüche, **dadurch gekennzeichnet, dass** das Stentgerüst (2) zur Verankerung der Herzklappenprothese (1) Zellen (30) am Ausströmtrakt (27) aufweist, die nach außen gebogen sind.

13. Herzklappenprothese nach einem der vorhergehenden Ansprüche, **dadurch gekennzeichnet, dass** das Stentgerüst (2) an einem umlaufenden Rand (310) eines Einströmtrakts (31) des Stentgerüsts (2) verkürzte Zellen (33) aufweist, deren Länge in Richtung der Achse (A') kürzer ist als die Länge von in der Umfangsrichtung (U) benachbarten Zellen (34), wobei die verkürzten Zellen (33) dazu konfiguriert sind, im expandierten und insplantierten Zustand des Stentgerüsts (2) im Bereich des Aortenpeaks (AP) der nativen Mitralklappe (M) zu liegen zu kommen.

14. Herzklappenprothese nach einem der vorhergehenden Ansprüche, **dadurch gekennzeichnet, dass** das Stentgerüst (2) expandierbar, vorzugsweise selbstexpandierend ausgebildet ist.

15. Herzklappenprothese nach einem der vorhergehenden Ansprüche, **dadurch gekennzeichnet, dass** die Herzklappenprothese (1) dazu konfiguriert ist mittels eines Katheters implantiert zu werden.
